(19) Europäisches Patentamt
European Patent Office
Office européen des brevets

(11) Numéro de publication: **0 274 314 B1**

(12) # FASCICULE DE BREVET EUROPEEN

(45) Date de publication du fascicule du brevet: **07.11.90**

(51) Int. Cl.⁵: **C07C 69/757**, C07C 69/743, A01N 53/00

(21) Numéro de dépôt: **87402823.6**

(22) Date de dépôt: **11.12.87**

(54) **Procédé de préparation du 2,2-diméthyl 3-formyl cyclopropane carboxylate de pentafluorophénylméthyle et son application à la synthèse de produits pesticides.**

(30) Priorité: **11.12.86 FR 8617332**

(43) Date de publication de la demande: **13.07.88 Bulletin 88/28**

(45) Mention de la délivrance du brevet: **07.11.90 Bulletin 90/45**

(84) Etats contractants désignés: **AT BE CH DE ES FR GB GR IT LI LU NL SE**

(56) Documents cités:
**EP-A- 109 909**
**EP-A- 217 342**
**EP-A- 269 514**
**EP-A- 0 050 534**
**EP-A- 0 107 570**

**Fujita M. et al. (Sagami Chemical Research Center) Tetrahedron Letters, vol. 27, No. 19, p. 2139-2142 (1986)**

(73) Titulaire: **ROUSSEL-UCLAF, 35, boulevard des Invalides, F-75007 Paris(FR)**

(72) Inventeur: **Tessier, Jean, 30, rue Jean Moulin, F-94300 Vincennes(FR)**
Inventeur: **Demoute, Jean-Pierre, 249 bis, rue de Rosny, F-93100 Montreuil-Sous-Bois(FR)**
Inventeur: **Cadiergue, Joseph, 6, rue Jules Princet, F-93600 Aulnay-Sous-Bois(FR)**

(74) Mandataire: **Tonnellier, Marie-José et al, 111, route de Noisy B.P. no 9, F-93230 Romainville(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

L'invention concerne un procédé de préparation du 2,2-diméthyl 3-formyl cyclopropane carboxylate de pentafluorophénylméthyle et son application à la synthèse de produits pesticides.

On connaissait déjà des produits pesticides préparés à partir du 2,2-diméthyl 3-formyl cyclopropane carboxylate de pentafluorophényl méthyle (cf Fujita Met Al Tetrahedron letters, vol. 27, N° 19, pages 2139–2142 (1986), cf EP-A 217 342); on vient de découvrir une nouvelle voie d'accès aux produits pesticides de formule:

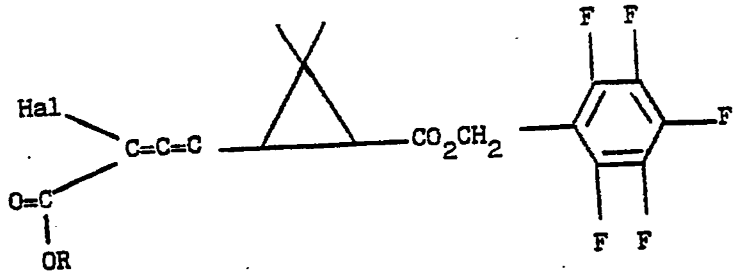

décrits dans le brevet européen 50534 ou le brevet d'invention français 2 536 389 mettant en œuvre le 2,2-diméthyl 3-formyl cyclopropane carboxylate de pentafluoro phényl méthyle.

L'invention a pour objet un procédé de préparation des composés de formule (I)):

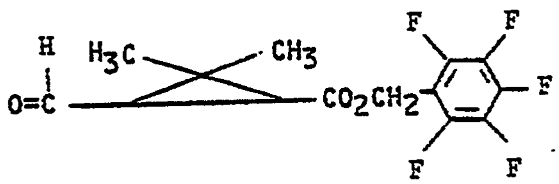

(I)

sous toutes les formes stéréoisomères possibles ainsi que les mélanges de ces stéréoisomères, caractérisé en ce que l'on soumet un composé de formule (II):

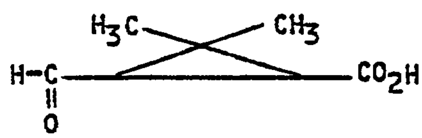

(II)

sous toutes les formes stéréoisomères possibles ainsi que les mélanges de ces stéréoisomères, à l'action d'un composé de formule (III):

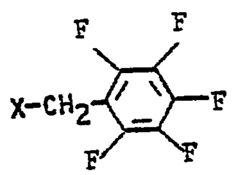

(III)

dans laquelle X représente un atome de fluor, de chlore, de brome ou d'iode, ou X représente un radical OZ dans lequel Z représente

un radical $-R_1SO_2$, ou un radical $(R_2O)_2\underset{\overset{\|}{O}}{P}-$,

$R_1$ et $R_2$ représentant un radical alcoyle renfermant de 1 à 8 atomes de carbone, en présence d'un agent basique pour obtenir le composé de formule (I) correspondant.

Les produits préparés peuvent ainsi être de configuration 1R, cis ou 1R, trans, 1S cis ou 1S trans, ou être des mélanges de 2, 3 ou 4 des diastéréoisomères possibles.

Lorsque Z représente un radical

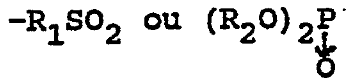

$R_1$ et $R_2$ représentent de préférence un radical méthyle, éthyle, n-propyle ou isopropyle.

Dans le procédé de l'invention, l'agent basique utilisé peut être un hydrure métallique, un alcoolate alcalin ou alcalino-terreux, un carbonate alcalin ou alcalino-terreux, une amine tertiaire, un hydroxyde métallique ou un amidure.

Dans un mode de réalisation préféré du procédé de l'invention, l'agent basique utilisé est un hydrure métallique comme l'hydrure de sodium.

L'invention a tout particulièrement pour objet, un procédé de préparation dans lequel le composé de formule (II) utilisé est l'acide 1R cis 2,2-diméthyl 3-formyl cyclopropane carboxylique en équilibre avec sa forme cyclique 1R, 5S, 6,6-diméthyl 4(R) hydroxy 3-oxabicyclo [3.1.0.] hexane-2-one; ce procédé conduit au composé de formule (I) dans lequel la copule cyclopropanique est de structure 1R, cis, c'est-à-dire le 1R, cis 2,2-diméthyl 3-formyl cyclopropane carboxylate de pentafluorophényl méthyle. La partie expérimentale exposée ci-après décrit une préparation de ce composé.

Les produits de formule (I) trouvent leur application dans la synthèse de produits pyréthrinoïdes doués de propriétés pesticides.

L'invention a également pour objet un procédé de préparation des composés de formule (V):

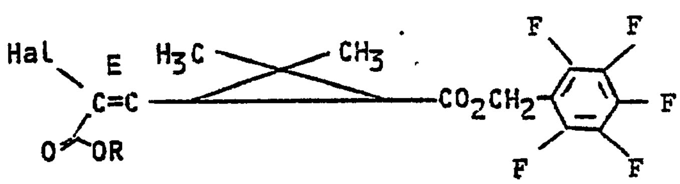

(V)

dans laquelle la géométrie de la double liaison est essentiellement E, et Hal représente un atome d'halogène, caractérisé en ce que:

a) on prépare le composé de formule (I):

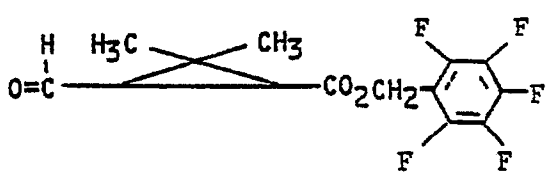

(I)

comme indiqué précédemment, puis

b) soumet le composé de formule (I) ainsi préparé à l'action d'un composé de formule (IV):

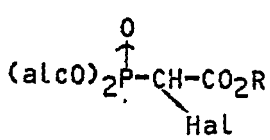

(IV)

dans laquelle alc représente un radical alcoyle renfermant de 1 à 8 atomes de carbone, Hal conserve sa signification précédente et R représente un radical alcoyle, linéaire, ramifié ou cyclique, saturé ou insaturé éventuellement substitué, pour obtenir le composé de formule (V) correspondant.

L'invention a notamment pour objet un procédé caractérisé en ce que l'on prépare d'abord le composé de formule ($I_a$):

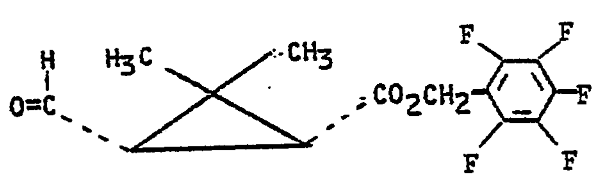

($I_a$)

dans laquelle la copule cyclopropanique est de structure 1R cis, puis soumet le dit composé de formule ($I_a$) à l'action d'un composé de formule ($IV_a$):

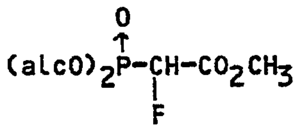

(IV$_a$)

dans laquelle alc a la signification indiquée ci-dessus pour obtenir le composé de formule (V$_a$) correspondant:

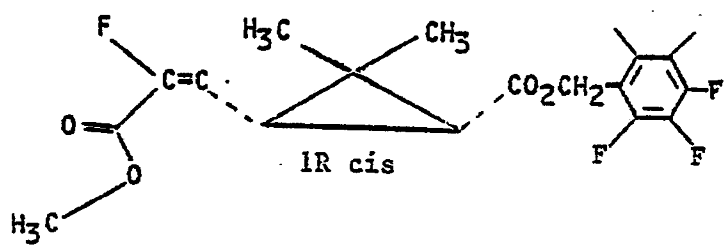

(V$_a$)

Dans un mode de réalisation préférée du procédé ci-dessus décrit, alc représente un radical méthyle, éthyle ou n-propyle.

La réaction entre le composé de formule (I) et le composé de formule (IV) a lieu de préférence au sein d'un éther oxyde tel que le tétrahydrofuranne, en présence de bromure de lithium et de diisopropylamine.

Les produits de formule (V) sont des produits connus, voir par exemple le brevet européen 50534, ou le brevet d'invention français 2 536 389.

Dans ces brevets, les produits de formule (V) peuvent être préparés par estérification de l'acide (VI):

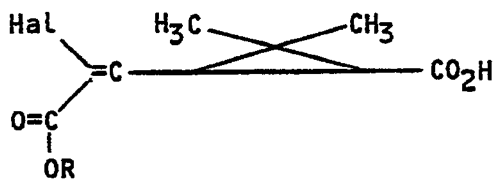

(VI)

par l'alcool pentafluorobenzylique, le produit de formule (VI) étant préparé par réaction de Wittig en faisant réagir le composé de formule (II), avec un phosphorane de formule:

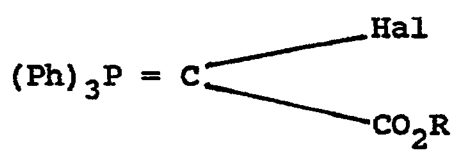

Le procédé de la présente invention permet d'obtenir les produits de formule (V) de géométrie E avec un rendement très supérieur à celui des brevets cités.

Le procédé de l'invention permet notamment d'obtenir le produit (V$_a$) ou 1Rcis(E) 2,2-diméthyl 3-[3-oxo 3-méthoxy 2-fluoro 1-propényl] cyclopropane carboxylate de pentafluorophényl méthyle produit présentant de bonnes propriétés pesticides.

Par rapport au procédé de préparation de ce produit connu jusqu'ici, les avantages du présent procédé sont les suivants:

– d'une part, le rendement en isomère de géométrie E est supérieur et
– d'autre part, l'isolement réalisable par simple cristallisation à partir du milieu réactionnel est beaucoup moins laborieux et a un caractère plus industriel.

L'exemple suivant illustre l'invention sans toutefois la limiter.

EXEMPLE: 1R, cis 2,2-diméthyl 3-formyl cyclopropane carboxylate de pentafluorophéanylméthyle.

On dissout 16,38 g de 1R, 5S 6,6-diméthyl 4-(R)-hydroxy 3-oxabicyclo [3.1.0.] hexane 2-one dans 750 cm$^3$ de tétrahydrofuranne, introduit lentement 5,52 g d'hydrure de sodium à 50% dans l'huile de vaseline, agite la suspension obtenue à 0, +5°C jusqu'à la fin du dégagement gazeux (environ 20 minutes), introduit en une seule fois une solution de 30 g de bromure de pentafluorobenzyle dans 120 cm$^3$ de diméthylformamide, agite le mélange réactionnel à 20°C pendant 16 heures, verse dans l'eau, extrait à l'éther isopropylique, concentre à sec par distillation sous pression réduite, chromatographie le résidu sur silice, sous pression, en éluant par un mélange d'hexane et d'acétate d'éthyle (9–1) et obtient 33 g de composé recherché (rendement 89%) Rf = 0,14.

Contrôles :

Spectre IR ($CHCl_3$)

>C = O 1734 $cm^{-1}$ , 1698 $cm^{-1}$

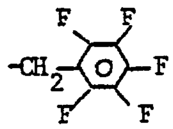

1658 $cm^{-1}$ , 1522 $cm^{-1}$ , 1508 $cm^{-1}$

Spectre RMN ($CDCl_3$)

| | |
|---|---|
| 1,3-1,5 ppm | H des méthyles géminés |
| 1,7-1,9 ppm | H en position 3 du cyclopropyle |
| 2,0-2,2 ppm | H en position 1 du cyclopropyle |
| 5,20-5,23-R,25 ppm | H du méthylène du pentafluorobenzyle |
| 9,65-9,75 ppm | H de l'aldéhyde. |

Application: 1R, cis (E) 2, 2-diméthyl 3-[3-oxo 3-méthoxy 2-fluoro 1-propényl] cyclopropane carboxylate de pentafluorophénylméthyle (composé A).

On mélange 80 $cm^3$ de tétrahydrofuranne, 7,82 g de bromure de lithium anhydre, introduit à −60°C en une seule fois 6,6 $cm^3$ de diisopropylamine, ajoute une solution de 6,84 g de 1R, cis 2,2-diméthyl 3-formyl cyclopropane carboxylate de pentafluorophénylméthyle (obtenu à l'exemple ci-dessus) 5,32 g de 0,0-diéthyl phosphonate de monofluoro acétate de méthyle et 80 $cm^3$ de tétrahydrofuranne, agite pendant 10 minutes à −60°C, verse le mélange réactionnel dans une solution aqueuse N d'acide chlorhydrique, extrait à l'éther, concentre à sec par distillation sous pression réduite, ajoute au résidu 50 $cm^3$ de méthanol, agite, ajoute 20 $cm^3$ d'eau, amorce, maintient à 0,+5°C pendant 1 heure et 30 minutes, essore les cristaux formés, sèche et obtient le produit recherché (100% en isomère E). F = 33,5°C.

Contrôles :

$[alpha]_D$ = + 10,5° (c = 1 % $CHCl_3$)

Spectre IR ($CHCl_3$)

>C = O 1730 $cm^{-1}$

C = C (E)

+ aromatique du type 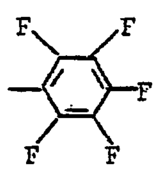 1658 $cm^{-1}$, 1522 $cm^{-1}$, 1508

Spectre RMN ($CDCl_3$)

| | |
|---|---|
| 1,3 ppm | H des méthyles géminés |
| 1,9 ppm | H en position 1 du cyclopropyle |
| 2,9 ppm | H en position 3 du cyclopropyle |
| 3,9 ppm | H du $CH_3$ du méthoxy |
| 5,2 ppm | H du méthylène du pentafluorobenzyle |
| 6,3 ppm | H éthylénique. |

Analyse: $C_{17}H_{14}F_6O_4$ = 396,29

| | C % | H % | F % |
|---|---|---|---|
| Calculé | 51,52 | 3,56 | 28,76 |
| Trouvé | 51,5 | 3,5 | 28,4 |

**Revendications**

1. Procédé de préparation des composés de formule (I):

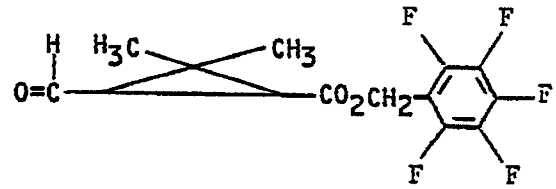

(I)

sous toutes les formes stéréoisomères possibles ainsi que les mélanges de ces stéréoisomères, caractérisé en ce que l'on soumet un composé de formule (II):

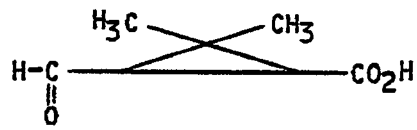

(II)

sous toutes les formes stéréoisomères possibles ainsi que les mélanges de ces stéréoisomères, à l'action d'un composé de formule (III):

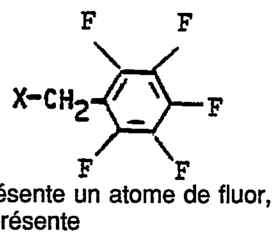

(III)

dans laquelle X représente un atome de fluor, de chlore, de brome ou d'iode, ou X représente un radical OZ dans lequel Z représente

$$\text{un radical } -R_1SO_2, \text{ ou un radical } (R_2O)_2\underset{O}{\underset{\downarrow}{P}}-,$$

$R_1$ et $R_2$ représentant un radical alcoyle renfermant de 1 à 8 atomes de carbone, en présence d'un agent basique pour obtenir le composé de formule (I) correspondant.

2. Procédé selon la revendication 1, caractérisé en ce que le composé de formule (II) utilisé est l'acide 1R, cis 2,2-diméthyl 3-formyl cyclopropane carboxyliqne en équilibre avec sa forme cyclique 1R, 5S, 6,6-diméthyl 4(R) hydroxy 3-oxabicyclo [3.1.0.] hexane-2-one.

3. Procédé de préparation des composés de formule (V):

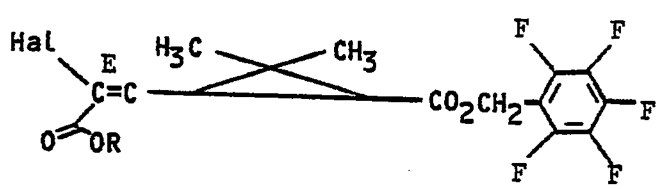

(V)

dans laquelle la géométrie de la double liaison est essentiellement E, et Hal représente un atome d'halogène, caractérisé en ce que:

a) on prépare le composé de formule (I):

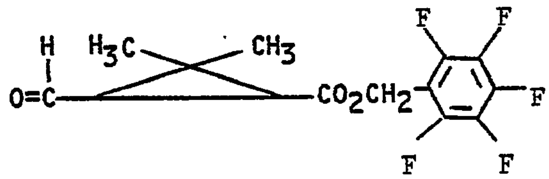 (I)

comme indiqué à la revendication 1 ou 2 puis,

b) soumet le composé de formule (I) ainsi préparé à l'action d'un composé de formule (IV):

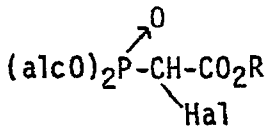 (IV)

dans laquelle alc représente un radical alcoyle renfermant de 1 à 8 atomes de carbone, Hal conserve sa signification précédente et R représente un radical alcoyle, linéaire, ramifié ou cyclique, saturé ou insaturé éventuellement substitué, pour obtenir le composé de formule (V) correspondant.

4. Procédé selon la revendication 3, caractérisé en ce que l'on prépare d'abord le composé de formule ($I_a$):

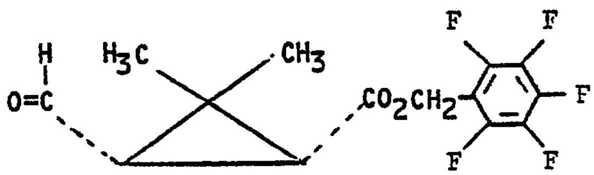 ($I_a$)

dans laquelle la copule cyclopropanique est de structure 1Rcis puis soumet le dit composé dé formule ($I_a$), à l'action d'un composé de formule ($IV_a$):

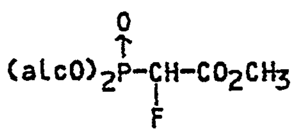 ($IV_a$)

dans laquelle alc a la signification indiquée ci-dessus pour obtenir le composé de formule ($V_a$) correspondant:

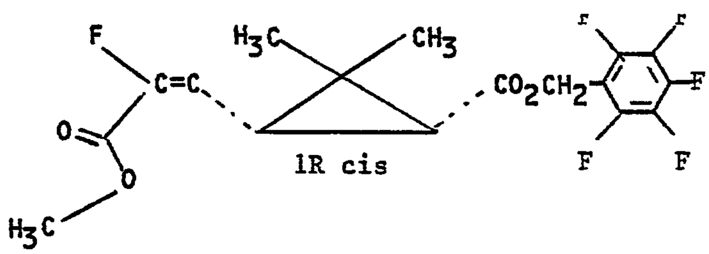 ($V_a$)

## Patentansprüche

1. Verfahren zur Herstellung von Verbindungen der Formel (I)

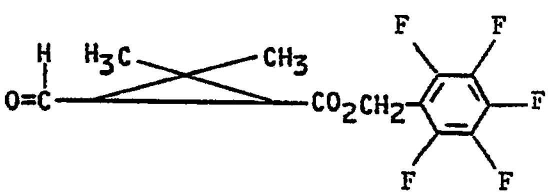 (I)

in sämtlichen ihrer möglichen stereoisomeren Formen sowie in Form von Gemischen dieser Stereoisomeren, dadurch gekennzeichnet, dass man eine Verbindung der Formel (II)

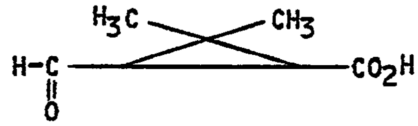

(II)

in sämtlichen ihrer möglichen stereoisomeren Formen sowie in Form von Gemischen dieser Stereoisomeren der Einwirkung einer Verbindung der Formel (III)

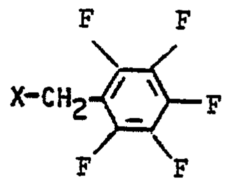

(III)

worin X für ein Fluor-, Chlor-, Brom- oder Jodatom steht oder X einen Rest OZ wiedergibt, worin Z für einen Rest

$-R_1SO_2$- oder einen Rest $(R_2O)_2\underset{\downarrow \atop O}{P}-$

steht, wobei $R_1$ und $R_2$ einen Alkylrest mit 1 bis 8 Kohlenstoffatomen bedeuten, in Gegenwart eines basischen Mittels unterzieht, um zu der entsprechenden Verbindung der Formel (I) zu gelangen.

2. Verfahren gemäss Anspruch 1, dadurch gekennzeichnet, dass die verwendete Verbindung der Formel (II) die 1R, cis-2,2-Dimethyl-3-formylcyclopropan-carbonsäure im Gleichgewicht mit ihrer cyclischen Form 1R, 5S-6,6-Dimethyl-4(R)-hydroxy-3-oxabicyclo[3.1.0]hexan-2-on ist.

3. Verfahren zur Herstellung der Verbindung der Formel (V)

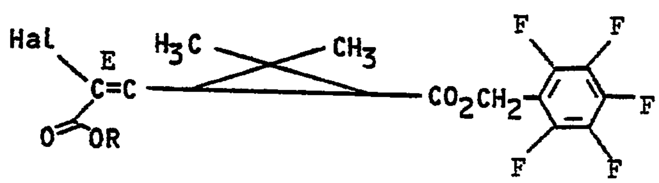

(V)

worin die Geometrie der Doppelbindung im wesentlichen die E-Geometrie ist und Hal ein Halogenatom bedeutet, dadurch gekennzeichnet, dass man

a) die Verbindung der Formel (I)

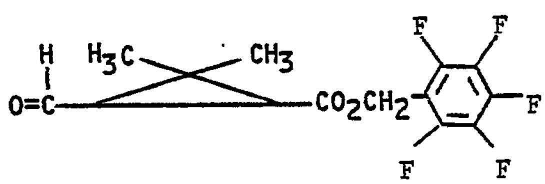

(I)

wie in Anspruch 1 oder 2 angegeben, herstellt und hiernach

b) die so hergestellte Verbindung der Formel (I) der Einwirkung einer Verbindung der Formel (IV)

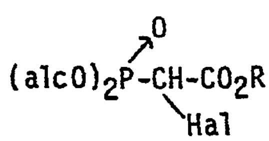

(IV)

unterzieht, worin alc einen Alkylrest mit 1 bis 8 Kohlenstoffatomen bedeutet, Hal die vorstehende Bedeutung besitzt und R für einen gesättigten oder ungesättingten, linearen, verzweigten oder cyclischen, gegebenenfalls substituierten Alkylrest steht, um zu der entsprechenden Verbindung der Formel (V) zu gelangen.

4. Verfahren gemäss Anspruch 3, dadurch gekennzeichnet, dass man zunächst die Verbindung der Formel ($I_a$)

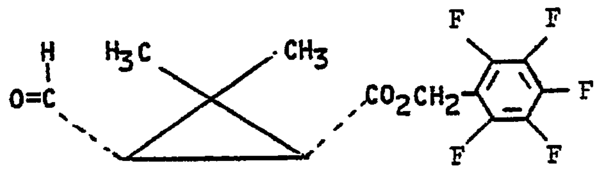

$(I_a)$

herstellt, worin die Cyclopropan-Verknüpfungskomponente die 1R, cis-Struktur besitzt, und hiernach die Verbindung der Formel ($I_a$) der Einwirkung einer Verbindung der Formel ($IV_a$)

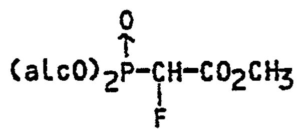

$(IV_a)$

worin alc die vorstehend angegebene Bedeutung besitzt, unterzieht, um zu der entsprechenden Verbindung der Formel ($V_a$)

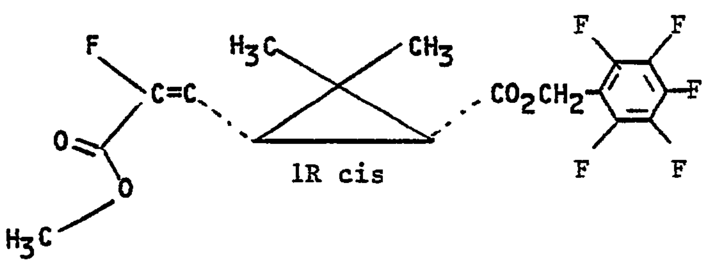

$(V_a)$

zu gelangen.

## Claims

1. Preparation process of compounds of formula (I):

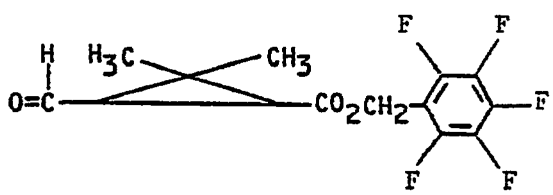

(I)

in all the possible stereoisomer forms as well as mixtures of these stereoisomers, characterized in that a compound of formula (II):

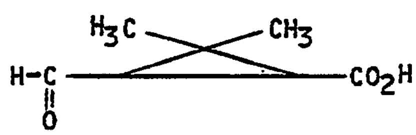

(II)

in all the possible stereoisomer forms as well as mixtures of these stereoisomers, is subjected to the action of a compound of formula (III):

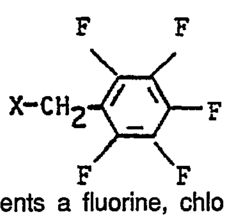

(III)

in which X represents a fluorine, chlorine, bromine or iodine atom, or X represents an OZ radical in which Z represents a

$-R_1SO_2$ radical, or a $(R_2O)_2\underset{\downarrow\atop O}{P}-$ radical,

$R_1$ and $R_2$ representing

an alkyl radical containing 1 to 8 carbon atoms, in the presence of a basic agent so as to obtain the corresponding compound of formula (I).

2. Process according to claim 1, characterized in that the compound of formula (II) used is 1R, cis-2,2-dimethyl-3-formyl-cyclopropane carboxylic acid in equilibrium with cyclic form 1R, 5S, -6,6-dimethyl-4(R)-hydroxy-3-oxabicycl [3.1.0.]-hexane-2-one.

3. Preparation process of compounds of formula (V):

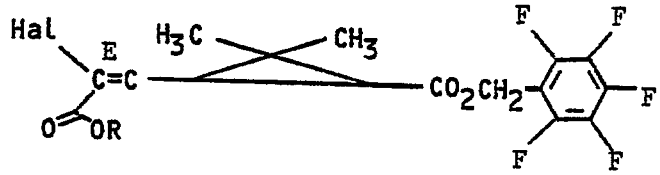

(V)

in which the geometry of the double bond is essentially E, and Hal represents a halogen atom, characterized in that:

a) the compound of formula (I):

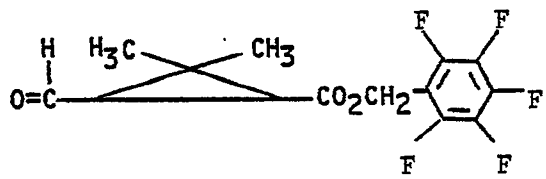

(I)

is prepared as indicated in claim 1 or 2 then,

b) the compound of formula (I) thus prepared is subjected to the action of a compound of formula (IV):

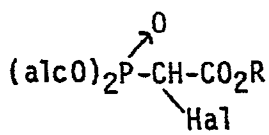

(IV)

in which alk represents an alkyl radical containing 1 to 8 carbon atoms, Hal retains its previous significance and R represents an optionally substituted, saturated or unsaturated, linear, branched or cyclic alkyl radical, so as to obtain the corresponding compound of formula (V).

4. Process according to claim 3, characterized in that the compound of formula ($I_a$), is prepared first:

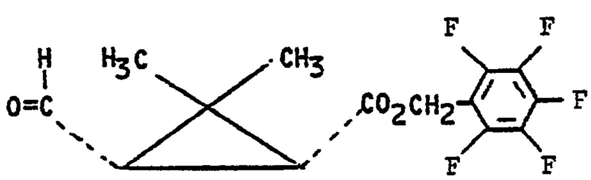

($I_a$)

in which the cyclopropane copula is of 1Rcis structure, then the said compound of formula ($I_a$) is subjected to the action of a compound of formula ($IV_a$):

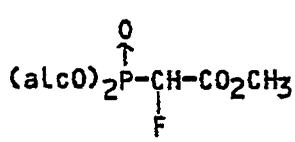

($IV_a$)

in which alk has the meaning indicated above so as to obtain the corresponding compound of formula ($V_a$).

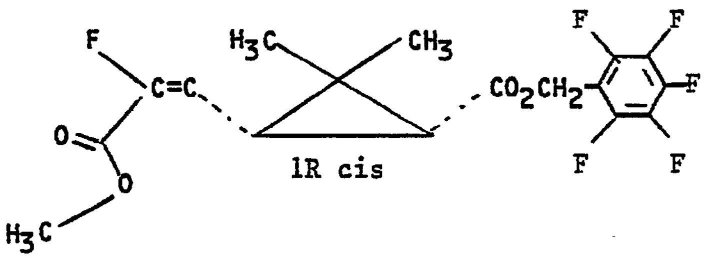

$(V_a)$